# EUROPÄISCHE PATENTSCHRIFT

(11) **EP 0 870 061 B1**
(45) Veröffentlichungstag und Bekanntmachung des Hinweises auf die Patenterteilung: **17.10.2001**
(21) Anmeldenummer: 96945752.2
(22) Anmeldetag: 27.11.1996
(51) Int. Cl.: C12Q 1/68

(54) **VERFAHREN ZUM NACHWEIS DER EXPRESSION VON CD95-LIGAND IN ZELLEN**
PROCESS FOR DETECTING THE EXPRESSION OF CD95 IN CELLS
PROCEDE POUR DECELER L'EXPRESSION DU LIGAND CD95 DANS DES CELLULES

(30) Priorität: 28.11.1995 DE 19544332
(43) Veröffentlichungstag der Anmeldung: 14.10.1998
(73) Patentinhaber: Deutsches Krebsforschungszentrum Stiftung des öffentlichen Rechts, 69120 Heidelberg (DE)
(72) Erfinder: DEBATIN, Klaus-Michael, D-69120 Heidelberg (DE); HERR, Ingrid, D-75015 Bretten (DE)
(74) Vertreter: Schüssler, Andrea, Dr.
(86) Internationale Anmeldenummer: DE9602274
(87) Internationale Veröffentlichungsnummer: WO9720067

(56) Entgegenhaltungen:
- EP-A- 0 675 200
- WO-A-91/02817
- 37TH ANNUAL MEETING OF THE AMERICAN SOCIETY OF HEMATOLOGY, SEATTLE, WASHINGTON, USA, DECEMBER 1-5, 1995. BLOOD 86 (10 SUPPL. 1). 163A. ISSN: 0006-4971, 15.November 1995, XP002035052 HERR I ET AL: "Development of a quantitative RT-PCR for the human APO-1 (FAS-CD95) ligand and monitoring of ligand expression in normal and malignant T cells."
- 37TH ANNUAL MEETING OF THE AMERICAN SOCIETY OF HEMATOLOGY, SEATTLE, WASHINGTON, USA, DECEMBER 1-5, 1995. BLOOD 86 (10 SUPPL. 1). 288A. ISSN: 0006-4971, 15.November 1995, XP002035053 DEBATIN K M ET AL: "Involvement of the APO-1 (FAS-CD95) system in T cell depletion in AIDS."
- LEUKEMIA, Bd. 9, Juli 1995, Seiten 1227-1232, XP002035054 PANAYIOTIDIS ET AL.: "Expression and function of the FAS antigen in B chronic lymphocytic leukemia and hairy cell leukemia"
- INTERNATIONAL IMMUNOLOGY, Bd. 6, Nr. 10, 1994, Seiten 1567-1574, XP002035055 TAKAHASHI ET AL: "Human FAS ligand: gene structure, chromosomal location and species specificity" in der Anmeldung erwähnt
- NATURE, Bd. 373, Februar 1995, LONDON GB, Seiten 438-441, XP002035056 DHEIN ET AL.: "Autocrine T-cell suicide by APO-1"
- CELL DEATH AND DIFFERENTIATION, Bd. 3, Juli 1996, Seiten 299-305, XP002035057 HERR ET AL.: "Monitoring of CD95 ligand expression in human T cells by quantitative RT-PCR"

## Beschreibung

Die vorliegende Erfindung betrifft ein Verfahren zum Nachweis der Expression von CD95-Ligand in Zellen.

Apoptose ist die Bezeichnung für den programmierten Zelltod. Dieser findet sich z.B. bei der Organentwicklung und Metamorphose, der Gewebeatrophie und Tumorregression. Apoptose ist mit einer Kondensation des Cytoplasmas, einem Verlust von Plasmamembranvilli, einer Segmentation des Kerns und extensivem Abbau chromosomaler DNA verbunden. Es hat sich gezeigt, daß Störungen der Apoptose bei verschiedenen Erkrankungen, wie AIDS, Autoimmunerkrankungen, Tumoren und Leukämien, auftreten. Bei AIDS scheint eine gesteigerte Apoptose für die starke Abnahme der CD4-T Zellen verantwortlich zu sein.

In aktivierten T-Zellen wie auch in anderen Zellen, findet sich ein mit CD95 bzw. APO-1 bezeichnetes Zelloberflächenprotein. An dieses Zelloberflächenprotein kann ein mit CD95-Ligand bzw. APO1-Ligand bezeichnetes lösliches oder Membrangebundenes Protein binden und die Induktion von Apoptose auslösen.

Das Ausmaß und der Verlauf von Apoptose sowie die diese verursachenden Moleküle können bisher nicht bestimmt werden. Dies wäre aber notwendig, um z.B. Verlaufskontrollen und geeignete Therapiemaßnahmen bei Patienten mit den zuvor genannten Erkrankungen durchführen zu können.

Debatin et al., Blood 86, No. 10, Suppl. 1, Abstr. Nr. 1139 beschreiben die Beteiligung des Apo-1 (Fas/CD95) Systems bei der T-Zell Verminderung bei AIDS

Herr et al., Blood 86, No. 10, Suppl. 1, Abstr. Nr. 639 beschreiben die Entwicklung einer quantitativen RT-PCR für den menschlichen Apo-1 (Fas/CD95) Liganden und das Verfolgen des Ligandenexpression in normalen und malignen T-Zellen.

Der vorliegenden Erfindung liegt somit die Aufgabe zugrunde, ein Mittel bereitzustellen, mit dem das Ausmaß bzw. der Verlauf von Apoptose bestimmt werden kann.

Erfindungsgemäß wird dies durch die Gegenstände in den Patentansprüchen erreicht.

Gegenstand der vorliegenden Erfindung ist somit ein Verfahren, mit dem CD95-Ligand, nachstehend mit CD95-L bezeichnet, in Zellen quantitativ bestimmt werden kann.

Die vorliegende Erfindung beruht auf der Erkenntnis der Anmelderin, daß Apoptose in T-Zellen durch eine erhöhte Menge von CD95-L versucht wird. Ferner fand die Anmelderin heraus, daß die Apoptoserate von der CD95-L Menge bestimmt wird.

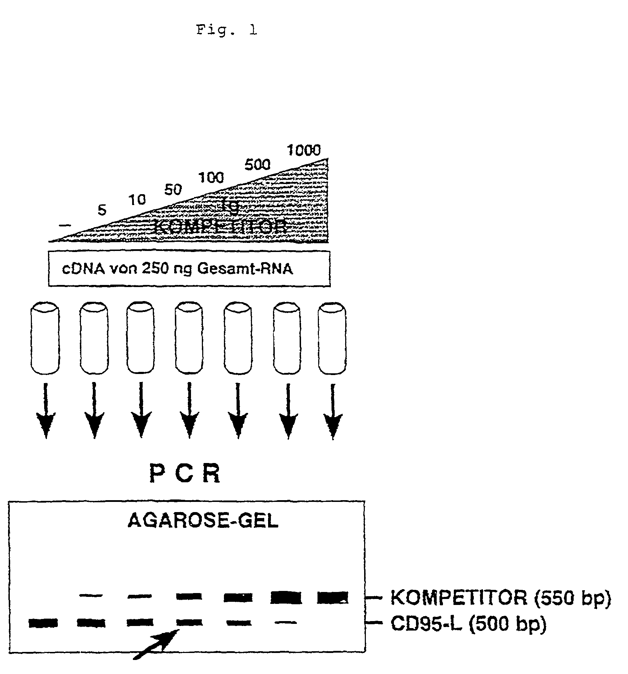

Das erfindungsgemäße Verfahren ist eine quantitative "Polymerase Chain Reaction" (PCR), mit der die Menge von CD95-L in Zellen verläßlich bestimmt werden kann. Im erfindungsgemäßen Verfahren wird die in der Zelle vorhandene Menge von CD95-L mRNA durch eine RT-PCR-Reaktion bestimmt. Bei der quantitativen PCR für CD95-L wird die Menge von CD95-L cDNA durch Zugabe eines CD95-L spezifischen DNA-Kompetitor-Fragments in die PCR-Reaktion titriert. Diesem Schritt voraus geht die vollständige reverse Transkription einer definierten Menge Gesamt-RNA in cDNA. Das Kompetitor-Fragment unterscheidet sich von der "Wildtyp" cDNA von CD95-L durch zusätzliche Basenpaare in der Mitte der Nukleotidsequenz, besitzt jedoch identische flankierende Sequenzen. Deshalb werden Wildtyp- und Kompetitor-Fragment von der gleichen Primer-Kombination erkannt und können zusammen in einer PCR vervielfältigt werden. Bedingt durch die identischen Primer-Bindungsstellen und die ähnlichen Nukleotidsequenzen unterliegen beide Fragmente der gleichen Amplifikationsrate. Der geringe Größenunterschied erlaubt jedoch die Auftrennung beider DNA-Spezies durch Gelektrophorese nach der PCR. Die Intensitäten der DNA-Banden auf dem Agarosegel reflektieren die ursprünglichen Mengen der cDNA, die in die PCR eingesetzt wurden. Wenn die Ausprägung der CD95-L und der Kompetitor-DNA-Bande auf dem Gel identisch ist, kann geschlossen werden, daß von Kompetitor und CD95-L die gleichen Ausgangskonzentrationen vorlagen. Die Menge der auf diese Weise titrierten CD95-L cDNA spiegelt die Menge der CD95-L mRNA in der Gesamt-RNA wieder, da die reverse Transkription vollständig verläuft. Das Schema der quantitativen PCR-Reaktion ist in Fig. 1 gezeigt.

Der quantitativen PCR liegt folgendes Reaktionsprinzip zugrunde. Zur Quantifizierung der CD95-L-Expressionsrate wird cDNA einer bestimmten Menge revers transkribierter Gesamt-RNA in PCR-Gefäße pipettiert. Jeder Probe mit Ausnahme der Negativkontrolle (-) wird Kompetitor-Fragment in unterschiedlicher Verdünnung, z.B. von 5 bis 10000 fg, zugefügt. Nach der PCR-Reaktion werden die amplifizierten DNA-Fragmente mit Ethidiumbromid behandelt und auf einem Agarosegel aufgetrennt. Die DNA wird durch Bestrahlen des Gels mit UV-Licht sichtbar gemacht. Es wird der Titrationspunkt (in Fig.1 mit einem Pfeil gekennzeichnet) bestimmt, zu dem sowohl die Bande der CD95-L DNA als auch die Bande der Kompetitor-DNA gleiche Intensitäten aufweisen. Ein gleiches Verhältnis beider Banden zeigt an, daß von der zu bestimmenden DNA und von der Kompetitor-DNA gleiche Mengen amplifiziert worden sind. Demzufolge ist die durch den Titrationspunkt bestimmte Menge an CD95-L cDNA (gleichzusetzen mit der Menge an mRNA) in der eingesetzten Menge Gesamt-RNA vorhanden gewesen.

Die für die PCR synthetisierten Primer basieren vorzugsweise auf der veröffentlichten Sequenz von CD95-L (vgl. Takahashi T. et al., int. Immunol. 6, (1984), 1547-1567). Die Durchführung der PCR erfolgt unter dem Fachmann bekannten Bedingungen.

Zur Amplifikation des CD95-L Fragmentes (ca. 500 bp) wie auch des Kompetitor-Fragments (ca. 550 bp) werden die folgenden Primer verwendet werden:
- Lᵤₚ: 5'-ATGTTTCAGCTCTTCCACCTACAGA-3'(25 mer, bindet an die Nukleotide 301 bis 325 der CD95-L Sequenz)
- L_{down}: 5'-CCAGAGAGAGCTCAGATACGTTGAC-3'(25 mer, bindet an die Nukeotide 775 bis 799 der CD95-L Sequenz)

Ferner können zur Herstellung des Kompetitor-Fragments (550 bp) z.B. folgende Primer verwendet werden:
- L_{upOV}: 5'-GGATCCGTACTACAGTGAAATTATGGAAGGGTATCCGAGTTCAGGAATTCCAGAGGCATGGACCTTGAGTTGGACTTGCC-3' (80-mer, bindet an die Nukleotide 451 bis 480)
- L_{dwOV}: 5'-CCTTCCATAATTTCACTGTAGTACGGATCCGAATGGGAAGACACCTATGGAATTGTCC-3' (58 mer, bindet an die Nukleotide 481 bis 508)

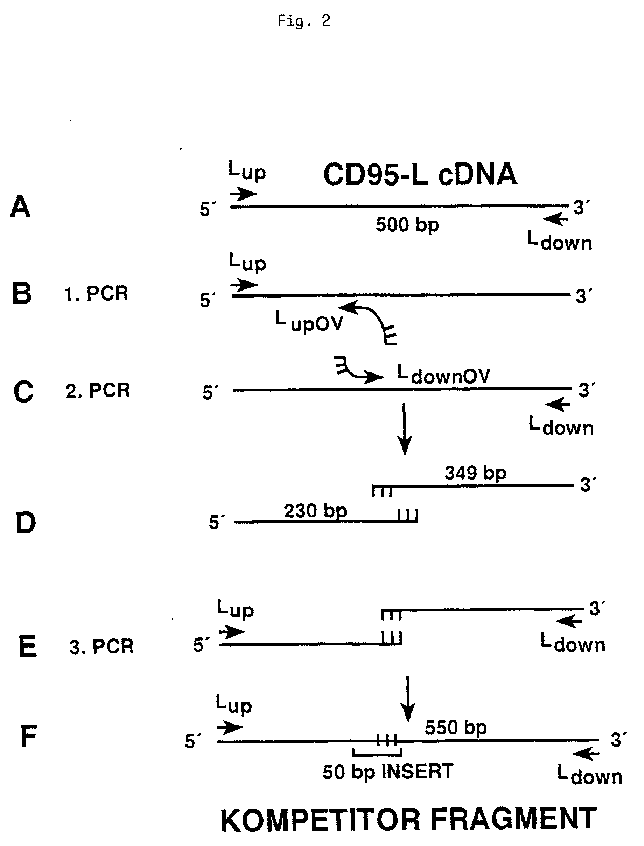

Das mutierte CD95-L Fragment, das in der PCR als Kompetitor-Fragment eingesetzt wird, wird durch Mutagenese hergestellt, vorzugsweise PCR-Mutagenese. Bei der PCR-Mutagenese dient als Matrize CD95-L cDNA, die z.B. der T-Zell-Linie CEM-S entstammt. Zwei Teile des Lᵤₚ/L_{down} PCR-Fragments werden amplifiziert, indem in einer PCR das Primerpaar Lᵤₚ/L_{upOV} (ergibt ein Fragment, das 230 bp lang ist) und in einer zweiten PCR das Primerpaar L_{down}/L_{downOV} (ergibt ein Fragment, das 349 bp lang ist) verwendet wird. Der Primer L_{upOV} enthält eine 50 bp lange überhängende Sequenz, die zu einer 30 bp langen überhängenden Sequenz des Primers L_{downOV} komplementär ist. Nach Aufreinigung werden die beiden Fragmente über ihre komplementären Regionen hybridisiert und als Matrize für eine erneute PCR-Amplifikation mit den Primern Lᵤₚ/L_{down} verwendet. Das Ergebnis ist ein mutiertes Fragment von CD95-L, das zusätzlich 50 Basen in der Mitte der Nukleotidsequenz enthält. Das Schema der Herstellung des Kompetitor-Fragments ist in Fig. 2 gezeigt.

Das erfindungsgemäße Verfahren auf der Basis einer Kompetition zwischen einem Mutantenfragment und einem Wildtypfragment ermöglicht die exakte Bestimmung des intrazellulären Levels von CD95-L in kleinen Probenmengen (1 bis 5 x 10⁶ Zellen)

Da die CD95/CD95-L vermittelte Apoptose offensichtlich an einer T-Zelldepletion bei einer AIDS-Erkrankung beteiligt ist, kann die quantitative Bestimmung der Expression von CD95-L zur Verlaufskontrolle und als Indikator für therapeutische Interventionen bei AIDS-Patienten herangezogen werden. Ferner eignet sich das erfindungsgemäße Verfahren in vitro zum "Drug-Screening" für potentielle Medikamente, die virusinduzierte, CD95-vermittelte Apoptose in T-Zellen blockieren können. In gleicher Weise kann bei Erkrankungen mit verminderter Apoptose (z.B. Leukämien) oder gesteigerter CD95-vermittelter Apoptose die quantitative Bestimmung der CD95-Liganden in Zellen zur Krankheitsdefinition, Verlaufskontrolle und zur pharmakologischen Beeinflußung der Ligandenexpression im "Drug Screening" eingesetzt werden. Als unmittelbares Anwendungsgebiet ergibt sich z.B. die Analyse der Wirkung von Medikamenten, wie Azothioprin, Cyclosporin A, Cyclophosphamid, Cortison und Methotrexat, die derzeit zur immunsuppressiven Behandlung von Autoimmunerkrankungen und Transplantatabstoßung eingesetzt werden.

### Kurze Beschreibung der Zeichnungen:

- Fig. 1: zeigt ein Schema der erfindungsgemäßen, quantitativen PCR für CD95-L.
- Fig. 2: zeigt ein Schema der Herstellung eines Kompetitor DNA-Fragments von CD95-L
(A) Das Wildtyp-Fragment und seine spezifischen Primer für die PCR-Amplifikation, Lᵤₚ und L_{down}, sind dargestellt.
(B) Für die 1. PCR werden Lᵤₚ und L_{upOV} benutzt. L_{uPOV} bindet in der Mitte der Sequenz und enthält eine 50 bp lange überhängende Sequenz am 5'-Ende.
(C) Für die zweite PCR werden L_{down} und L_{downOV} verwendet. Die letzten 30 bp am 5'-Ende von L_{downOV} sind überhängend und komplementär zu den letzten 30 bp am 5'-Ende von L_{upOV}. Die komplementären Regionen sind bürstenartig gezeichnet.
(D) zeigt die PCR-Produkte aus der ersten und zweiten PCR, ein 349 und 230 bp langes Stück DNA. Nach Aufreinigung werden beide Fragmente über ihre komplementären Regionen miteinander hybridisiert.
(E) Die in (D) hybridisierten Fragmente werden als Matrize für eine dritte PCR-Reaktion verwendet, bei der wieder Lᵤₚ und L_{down} als Primer benutzt werden.
(F) Das Resultat ist ein mutiertes Fragment von CD95-L, das ein 50 bp langes Insert in der Mitte der Sequenz trägt.

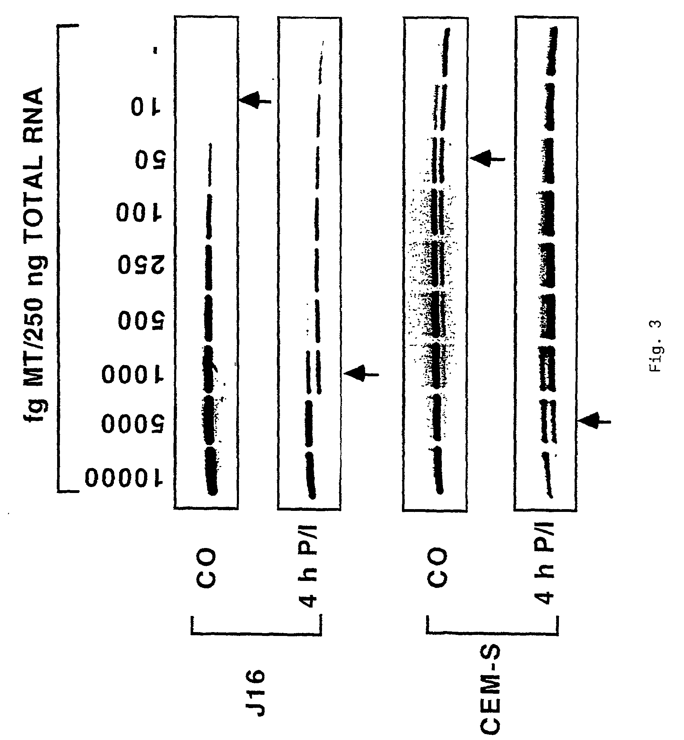

- Fig. 3: zeigt eine konstitutive und induzierte Expression von CD95-L mRNA in lymphoiden Zellen

Die vorliegende Erfindung wird durch das folgende Beispiel erläutert:

### Beispiel: Bestimmung von CD95-L in lymphoiden Zellen

Die lymphoiden T-Zellen J16 und CEM-S wurden jeweils in üblichem Medium kultiviert bzw. mit PMA (5 ng/ml) und lonomycin (2 *µ*g/ml) für 4 bzw. 24 Stunden behandelt. Aus den Zellen wurde Gesamt-RNA nach der Methode von Chomczynski, P. und Sacchi, N., Anal. Biochem., 162, (1987), 156-159 gewonnen und mit Hilfe von AMV-ReverserTranskriptase (Amersham, Braunschweig) und Oligo-dT16 Primern (Promega, Heidelberg) in cDNA umgeschrieben. Eine 20 *µ*l Reaktion enthielt 1x AMV-Puffer (50 mM Tris-HCI, pH 8,3; 8 mM MgCl₂; 50 mM NaCI; 1 mM DTT), 250 *µ*M eines jeden dNTP (Pharmacia, Freiburg), 0,5 *µ*M Oligo-dT₁₆; 0,6 U/*µ*l RNase-Inhibitor (Amersham, Braunschweig); 0,75 U/*µ*l AMV-reverse Transkriptase; 50 ng/*µ*l Gesamt-RNA (vor Zugabe 5 Minuten bei 65°C denaturiert). Die reverse Transkription wurde für 45 Minuten bei 42°C durchgeführt und die Reaktion wurde anschließend durch Erhitzen für 5 Minuten auf 94°C beendet. Da alle Reagenzien im Überschuß vorhanden waren, kann davon ausgegangen werden, daß die eingesetzte RNA vollständig zu cDNA umgechrieben worden ist.

Zur Quantifizierung der CD95-L Expressionsrate wurde cDNA von je 250 ng revers transkribierter Gesamt-RNA in PCR-Gefäße pipettiert. Jeder Probe mit Ausnahme der Negativkontrolle (-) wurde Kompetitor-Fragment, wie vorstehend beschrieben, in unterschiedlicher Verdünnung von 10 bis 10000 fg zugegeben. Das Reaktionsgemisch umfaßte: 50 *µ*l, enthaltend cDNA (= 250 ng revers transkribierte Gesamt-RNA), 1x PCR-Puffer (10 mM Tris-HCl, pH 8,8; 50 mM KCI, 0,08 % NP40), 1 mM MgCl₂, 200 *µ*M dNTPs, 0,5 *µ*M Primer und 1,12 U/Gefäß Taq-Polymerase (MBI/Fermentas). Die Amplifikation wurde in einem Robozykler (Stratagene) oder einem Biozym-Thermozykler unter Verwendung der Lᵤₚ- und L_{down}-Primer, wie vorstehend beschrieben, durchgeführt. Die Reaktionsbedingungen waren 35 Sek. bei 94°C, 90 Sek. bei 56°C und 120 Sek. bei 72°C über 36 Zyklen. 20 *µ*l der PCR-Reaktion wurden durch Elektrophorese auf einem 2% Agarose-Gel aufgetrennt und nach Ethidiumbromidanfärbung durch UV-Licht sichtbar gemacht. Auf dem Gel wurde der Punkt bestimmt, an dem sowohl die Bande der CD95-L DNA als auch die Bande der Kompetitor DNA gleiche Intensitäten aufwiesen. Das Gel ist in Fig. 3 gezeigt. Gleiche Bandenintensitäten, welche identische Mengen Wildtyp- und Kompetitorfragment anzeigen, sind durch Pfeile gekennzeichnet.

Es zeigte sich, daß unbehandelte J16 Zellen 10 fg CD95-L mRNA/250 ng Gesamt-RNA und unbehandelte CEM-S Zellen 50 fg CD95-L mRNA/250 ng Gesamt-RNA exprimierten. Eine PMA/lonomycin-Behandlung steigerte die Expression von CD95-L mRNA auf 1000 fg/250 ng Gesamt-RNA bei J16-Zellen und auf 5000 fg/250 ng Gesamt-RNA in CEM-S Zellen (vgl. Tabelle 1).

Vorstehende Daten zeigen, daß mit dem erfindungsgemäßen Verfahren CD95-L quantitativ bestimmt werden kann. Damit kann das Ausmaß und/oder der Verlauf von Apoptose, insbesondere bei Erkrankungen, wie AIDS, bestimmt werden.

**TABELLE 1**

| EXPRESSION VON CD95-L mRNA (fg/250 ng GESAMT-RNA) IN LYMPHOIDEN ZELL-LINIEN | | | | |
|---|---|---|---|---|
| | | CO | 4 h P/I | 24 h P/I |
| T-ZELL-LINIEN | J 16 | 10 | 1000 | 1000 |
| | CEM-S | 50 | 5000 | 250 |

## Patentansprüche

1. Verfahren zur quantitativen Bestimmung von CD95-Ligand, umfassend die folgenden Verfahrensschritte:
(a) Isolierung von Gesamt-RNA aus Zellen
(b) Umschreibung der Gesamt-RNA von (a) in cDNA durch reverse Transkription, und
(c) Amplifikation der cDNA von (b) und eines CD95-Ligand-Kompetitor-Fragments durch CD95-Ligand spezifische Primer in einer PCR-Reaktion, wobei
die CD95-Ligand spezifischen Primer die folgende Sequenz haben:
Lᵤₚ 5'-ATGTTTCAGCTCTTCCACCTACAGA-3'
L_{down} 5'-CCAGAGAGAGCTCAGATACGTTGAC-3'

2. Verfahren nach Anspruch 1, **dadurch gekennzeichnet, daß** die Zellen von einer etablierten Zellinie oder einem Patienten stammen.

3. Verfahren nach Anspruch 2, **dadurch gekennzeichnet, daß** die Zellen lymphoide T-Zellen sind.

4. Verwendung des Verfahrens nach Anspruch 1 zur Bestimmung des Ausmaßes und/oder Verlaufs von Apoptose.

5. Verwendung des Verfahrens nach Anspruch 4, wobei die Bestimmung bei Zellen eines AIDS-Patienten erfolgt.

## Claims

1. A process for the quantitative determination of CD95 ligand, comprising the following processing steps:
(a) isolation of whole RNA from cells,
(b) transcription of the whole RNA from (a) into cDNA by reverse transcription, and
(c) amplification of the cDNA from (b) and a CD95 ligand competitor fragment by CD95 ligand-specific primers in a PCR reaction, wherein
the CD95 ligand-specific primers have the following sequences:
Lᵤₚ 5'-ATGTTTCAGCTCTTCCACCTACAGA-3'
L_{down} 5' -CCAGAGAGAGCTCAGATACGTTGAC-3'

2. The process according to claim 1, **characterized in that** the cells originate from an established cell line or a patient.

3. The process according to claim 2, **characterized in that** the cells are lymphoid T cells.

4. Use of the process according to claim 1 for determining the extent and/or course of apoptosis.

5. Use of the process according to claim 4, wherein the determination is made with the cells of an AIDS patient.

## Revendications

1. Procédé pour la détermination quantitative de ligand CD95, comprenant les étapes opératoires suivantes :
(a) isolement d'un ARN complet à partir de cellules
(b) transcription de l'ARN complet de (a) en ADNc par transcription inverse et
(c) amplification de l'ADNc de (b) et d'un fragment de compétition de ligand CD95 au moyen d'amorces spécifiques du ligand CD95 lors d'une réaction d'amplification en chaîne par polymérase,
dans lequel les amorces spécifiques du ligand CD95 ont la séquence suivante :
L_{vers le haut} 5'-ATGTTTCAGCTCTTCCACCTACAGA-3'
L_{vers le bas} 5'-CCAGAGAGAGCTCAGATACGTTGAC-3'.

2. Procédé selon la revendication 1, **caractérisé en ce que** les cellules proviennent d'une lignée cellulaire établie ou d'un patient.

3. Procédé selon la revendication 2, **caractérisé en ce que** lea cellules sont des cellules T lymphoïdes.

4. Utilisation du procédé selon la revendication 1 pour la détermination de l'ampleur et/ou du déroulement d'une apoptose.

5. Utilisation du procédé selon la revendication 4, dans laquelle la détermination se fait avec des cellules d'un patient atteint du SIDA.
